# EUROPÄISCHE PATENTSCHRIFT

(11) **EP 3 584 595 B1**
(45) Veröffentlichungstag und Bekanntmachung des Hinweises auf die Patenterteilung: **20.10.2021**
(21) Anmeldenummer: 18178531.2
(22) Anmeldetag: 19.06.2018
(51) Int. Cl.: G01R 33/483, G01R 33/563, G01R 33/567

(54) **VERFAHREN ZUM ERSTELLEN VON ANGIOGRAPHISCHEN MR-BILDERN MITTELS GEKRÜMMTER BILDGEBUNGSSCHICHT-PROFILE**
METHOD OF MR-ANGIOGRAPHY USING CURVED IMAGING SLICES
PROCÉDÉ D'ANGIOGRAPHIE PAR RÉSONANCE MAGNÉTIQUE À L'AIDE DE TRANCHES D'IMAGERIE COURBÉES

(43) Veröffentlichungstag der Anmeldung: 25.12.2019
(73) Patentinhaber: Siemens Healthcare GmbH, 91052 Erlangen (DE)
(72) Erfinder: Graessner, Joachim, 25474 Bönningstedt (DE)

(56) Entgegenhaltungen:
- US-A- 5 755 666
- US-A1- 2012 016 224
- US-A1- 2012 249 139
- US-A1- 2012 262 171
- US-B1- 6 249 694
- BOERNERT P ET AL: "CURVED SLICE IMAGING", MAGNETIC RESONANCE IN MEDICINE, JOHN WILEY & SONS, INC, US, Bd. 36, Nr. 6, 1. Dezember 1996 (1996-12-01), Seiten 932-939, XP000636875, ISSN: 0740-3194

## Beschreibung

Die vorliegende Erfindung betrifft ein Verfahren zum Erstellen von angiographischen MR-Bildern, welche auf in ein Bildgebungsvolumen einfließenden ungesättigten Spins beruhen. Weiter werden eine zugehörige MR-Anlage, ein Computerprogrammprodukt und ein elektronisch lesbarer Datenträger bereitgestellt.

Bei der inflow-basierten MR-Angiographie (MRA) wird die Sensitivität des MR-Signals in Bezug auf die Bewegung von Spins verwendet, um Bilder von Gefäßen, insbesondere von Arterien und gelegentlich der Venen, zu erstellen. Bei Darstellung der Blutgefäße in der Peripherie, insbesondere der Becken-Bein-Abstrombahn oder dem Arm-Hand-Abschnitt, und auch in anderen Körperregionen kommen Nicht-Kontrastmittel (non-KM) - Verfahren der Magnet-Resonanz-Tomographie (MRT) zum Einsatz, die durch transversale Daten-Akquisition mit und ohne EKG Triggerung den Einstrom von frischem ungesättigtem Blut für eine hyperintense Darstellung der Gefäße ausnutzen.

Durch diskrete oder kontinuierliche Verschiebung der Position der gemessenen Bildgebungsschichten, zum Teil auch mit Overlap (dt. Überlappung), wird ein größerer Bereich komplett erfasst bevor durch Verschiebung der Tischposition dieser Vorgang erneut beginnt. Synonyme für derartige Verfahren, welche auf in ein Bildgebungsvolumen einfließenden ungesättigten Spins beruhen, sind inflow-basierte (dt. einflußbasierte) MR-Bildgebungsverfahren, Time-of-Flight (ToF) Techniken, oder Einstrom-Techniken. Ein Verfahren, welches in den letzten Jahren besondere Bedeutung erlangt hat, ist das QISS (Quiescent Intervall Single Shot) -Verfahren, ein EKGgetriggertes MRA-Verfahren ohne Kontrastmittelgabe, bei welchem die Bildakquisition mit dem Herzsignal des Patienten synchronisiert wird. Zunächst wird dabei die Signalintensität des stationären Gewebes innerhalb einer in Bezug auf eine Koordinatenachse der MR-Anlage transversalen Akquisitionsschicht, mittels eines nichtselektiven Sättigungs-RF-Impulses reduziert. Dieser Magnetisierungspräparation folgt ein Ruheintervall - das sogenannte Quiescent Interval (QI) - während dessen keine RF-Impuls-Anregung stattfindet. Das QI liegt hierbei idealerweise im Moment des maximalen systolischen Blutflusses in der Ausleseschicht. Die eigentliche Bildakquisition erfolgt während der Diastole bei langsamer Blutströmung. Das QISS-Verfahren ist beispielsweise aus der Druckschrift "Nonenhanced peripheral MR-angiography (MRA) at 3 Tesla: evaluation of quiescent-interval single-shot MRA in patients undergoing digital subtraction angiography." von Moritz Wagner et al. in The International Journal of Cardiovascular Imaging 31.4 (2015): 841-850 bekannt.

Herkömmliche inflow-basierte Verfahren der MR-Angiographie sind jedoch an anatomischen Positionen fehlerbehaftet, an welchen Blutgefäße nicht orthogonal zu einer transversalen Bildgebungsschicht, d.h. nicht parallel zu einer Koordinatenachse der MR-Anlage, verlaufen. Ändern die Gefäße ihren Verlauf in Bezug auf die transversale Akquisitionsschicht von einem orthogonalen Verlauf zu einem lateralen Verlauf, so weisen sie zumindest teilweise einen zur Bildgebungsschicht parallelen Verlauf auf. Dabei kommt es an diesen anatomischen Positionen zu einer weniger effektiven Füllung des Gefäßabschnittes mit ungesättigten einströmenden Spins, was zu einer hypointensen bis auslöschenden Darstellung des Gefäßabschnittes führt. Fehldiagnosen oder nicht diagnostizierbare Aufnahmen, in welchen beispielsweise Gefäßverschlüsse nicht erkannt werden können, sind die Folge davon. Man spricht bei diesem Phänomen auch von Inplane-Flow-Artefakten.

Besonders anfällig für diesen Effekt ist die Trifurkation unterhalb des Knies und partiell auch die Bifurkation im Beckenbereich. Eine Darstellung der Nierenarterien mit nahezu 90 Grad Abgängen der Gefäße ist bislang mit Einstromtechniken nicht möglich. Die einzige Möglichkeit, diese Inplane-Flow-Artefakte zu minimieren, bestand bislang in einer Reduktion der Schichtdicke, einer zusätzlichen mehr orthogonalen Messung des interessierenden Bereiches oder in einer Verlängerung der TR-Zeit, d.h. der Inflowzeit, was jeweils zu längerer Messzeit führte und bei der dünneren Schichtdicke auch ein geringeres Signal-Rausch-Verhältnis (SNR) zum Nachteil hat. Entsprechend müssen wegen der beschriebenen Nachteile der herkömmlichen MR-Verfahren in den kritischen Regionen oft zusätzlich hochaufgelöste Scans mit dünneren Bildgebungsschichten durchgeführt werden, beispielsweise mittels des High Resolution Thin Slice Protokolls, was zu einem erhöhten Zeit- und Personalaufwand, und damit erhöhten Untersuchungskosten führt. Ein manuelles Markieren der kritischen Regionen und ein Durchführen zusätzlicher hochaufgelöster Scans ist ein zeitraubendes und schwieriges Verfahren, das nur von geschulten Bedienpersonen durchgeführt werden kann.

Aus der Druckschrift US 5,755,666 A ist ein Magnetresonanzverfahren zum Abbilden eines gekrümmten Abschnitts eines Körpers bekannt, wobei in dem gekrümmten Abschnitt des Körpers durch 2DRF- oder 3DRF-Impulsfolgen und temporäre magnetische Gradientenfelder MR-Signale erzeugt werden, und wobei aus den empfangenen MR-Signalen ein Bild des gekrümmten Abschnitts unter Verwendung linearer Transformationen rekonstruiert wird.

Aus der Druckschrift "Curved slice imaging" von Peter Börnert und Tobias Schäffter in Magnetic resonance in medicine 36.6 (1996): 932-939, ist ein MRT-Bildgebungsverfahren mit gekrümmten Bildgebungsschichten bekannt, wobei durch 2DRF- oder 3DRF-Impulse beliebig definierte gekrümmte dreidimensionale Schichtprofile ermöglicht werden. Dabei werden die 2DRF- oder 3DRF-Impulse angepasst, um die Transversalmagnetisierung eines zuvor definierten gekrümmten Scheibenprofils in einem 3D-Raum anzuregen oder neu zu fokussieren.

Aus der Druckschrift US 2012/0249139 A1 ist ein MRT-Bildgebungsverfahren bekannt, wobei Bildgebungsschichten mit gekrümmten Schichtprofilen unter Verwenden von Gradientenkodierung nicht-linearere Gradienten angeregt werden, und wobei Rechteckimpulse zur Anregung mit sorgfältig ausgelegten Gradientenfeldern verwendet werden.

Aus der Druckschrift US 6,249,694 B1 ist ein peripheres MR-Angiographieverfahren zum Abbilden einer Arterie oder eines anderen Gefäßes bekannt, wobei MR-Daten einer Vielzahl von entlang des Gefäßes beabstandeten Scanstationen erfasst werden, wobei intravenös ein Kontrastmittel injiziert wird, um einen Bolus bereitzustellen, und wobei aus einer gekrümmten Bildgebungsschicht MR-Daten erfasst werden.

Weiterhin ist aus der Druckschrift US 2012/262171 A1 ein Verfahren zur Akquisition von MR-Daten aus gekrümmten Schichten und aus der Druckschrift US 2012/016224 A1 ein Verfahren zur Akquisition von MR-Daten aus einer Körperregion, welche ein fließendes Medium enthält, bekannt.

Es besteht daher Bedarf nach einem verbesserten Verfahren zum Erstellen von angiographischen MR-Bildern, welche auf dem Effekt der in ein Bildgebungsvolumen einfließenden ungesättigten Spins beruht, welches Gefäße einer Untersuchungsperson in einer MR-Anlage, die nicht parallel zu einer Koordinatenachse der MR-Anlage verlaufen, zuverlässig und fehlerfrei abbilden kann.

Diese Aufgabe wird mit den Merkmalen der unabhängigen Ansprüche gelöst. In den abhängigen Ansprüchen sind weitere Ausführungsformen der Erfindung beschrieben.

### Zusammenfassung

Gemäß einem ersten Aspekt der Erfindung wird ein Verfahren nach Anspruch 1 zum Erstellen von angiographischen MR-Bildern, welche auf in ein Bildgebungsvolumen einfließenden ungesättigten Spins beruhen, bereitgestellt. Dabei verlaufen darzustellende Gefäße, oder zumindest Gefäßabschnitte von darzustellenden Gefäßen, einer Untersuchungsperson, welche sich zur Untersuchung in einer MR-Anlage befindet, in nicht parallel zu einer Koordinatenachse der MR-Anlage, insbesondere zu der Z-Koordinatenachse der MR-Anlage. Einige Gefäßabschnitte können orthogonal, oder im Wesentlichen orthogonal, zu der Koordinatenachse der MR-Anlage verlaufen. Für den Fachmann ist dabei klar, dass nicht der exakt orthogonale Verlauf der Gefäßabschnitte erforderlich ist, sondern ein Verlauf mit derart hohem orthogonalem Anteil, dass Inplane-Flow-Artefakte auftreten können.

In einem ersten Schritt wird die Kernmagnetisierung in mindestens einer ersten Bildgebungsschicht der Untersuchungsperson zum Erzeugen von MR-Signalen angeregt. Die mindestens eine erste Bildgebungsschicht umfasst dabei die Gefäße, welche durch das bildgebende Verfahren dargestellt werden sollen zumindest teilweise. Die Bildgebungsschicht umfasst damit insbesondere auch solche Gefäßabschnitte, die nicht parallel zu einer Koordinatenachse der MR-Anlage verlaufen, insbesondere solche, die orthogonal zu der Koordinatenachse der MR-Anlage verlaufen. In einem weiteren Schritt werden die MR-Signale aus der mindestens einen ersten Bildgebungsschicht zum Erstellen von angiographischen MR-Bildern der Gefäße empfangen.

Dabei weist die mindestens eine erste Bildgebungsschicht ein gekrümmtes Schichtprofil auf. In anderen Worten, die mindestens erste Bildgebungsschicht weist bezüglich der Koordinatenachse der MR-Anlage gekrümmte Oberflächen auf.

Derartige Gefäßabschnitte, deren Verlauf bezüglich der Koordinatenachse einen hohen orthogonalen Anteil haben, verlaufen in herkömmlichen ebenen transversalen Bildgebungsschichten mit entsprechendem hohem Anteil parallel zu der Bildgebungsschicht. Sie verlaufen daher innerhalb der Bildgebungsschicht teilweise parallel zu der Bildgebungsschicht, oder über einen im Vergleich zu orthogonalen Gefäßen weiteren Bereich hinweg innerhalb der Bildgebungsschicht, wodurch Inplane-Flow-Artefakte begünstigt werden.

Durch das gekrümmte Schichtprofil, d.h. die Krümmung, der erfindungsgemäßen Bildgebungsschicht in inflow-basierten MR-Angiographieverfahren verlaufen Gefäßabschnitte, deren Verlauf nicht parallel zu der Koordinatenachse ist, senkrechter, d.h. mit einem höheren orthogonalen Flußanteil, zu der Bildgebungsschicht mit gekrümmtem Schichtprofil. In einem Extremfall schneiden Gefäßabschnitte, welche senkrecht zur Koordinatenachse verlaufen, und in herkömmlichen Einflußverfahren vollständig parallel und innerhalb der transversalen Bildgebungsschicht verlaufen, die erfindungsgemäße Bildgebungsschicht in einem steilen Winkel, so dass Inplane-Flow-Artefakte vermieden werden können. Ein manuelles Markieren solch kritischer Regionen und ein Durchführen zusätzlicher hochaufgelöster Scans kann somit vermieden werden. In anderen Worten kann durch die optimierten Anregungsprofile der Bildgebungsschicht Messzeit gespart werden und die Zuverlässigkeit der angiographischen Darstellung erhöht werden, da gekrümmte Schichtprofile einen orthogonaleren Inflow des Blutes und damit ein höheres Signal ermöglichen.

Dadurch wird ein verbessertes Verfahren zum Erstellen von inflow-basierten angiographischen MR-Bildern bereitgestellt, welches Gefäßabschnitte einer Untersuchungsperson, die nicht parallel zu einer Koordinatenachse einer MR-Anlage verlaufen, zuverlässig und fehlerfrei abbildet. Das Verfahren benötigt dadurch einen geringeren Zeit- und Personalaufwand, und kann dadurch bei geringeren Untersuchungskosten durchgeführt werden.

Das Anregen der Kernmagnetisierung in der mindestens einen ersten Bildgebungsschicht kann unter Verwendung eines linearen Gradientensystems der MR-Anlage erfolgen. Das lineare Gradientensystem, kann ein Standard-Gradientensystem sein, welches streng lineare Gradientenfelder in allen drei Raumrichtungen aufweist, und welches in Kombination mit einer 1,2- oder Mehrkanal-Hochfrequenzanregung zur Anregung der Kernspins eingesetzt wird.

Durch die Anregung der Kernspins unter Verwendung des linearen Gradientensystems kann eine beliebig geformte Bildgebungsschicht, beispielsweise mit gleichmäßiger Schichtdicke, realisiert werden, wobei keine nicht-linearen Charakteristiken einer Sendespule berücksichtig werden müssen.

Das gekrümmte Schichtprofil kann eines von einem parabelförmigen, hyperbelförmigen, V-förmigen oder pfeilförmigen Schichtprofil sein. Das gekrümmte Schichtprofil kann symmetrisch zur Koordinatenachse der MR-Anlage verlaufen. Das gekrümmte Schichtprofil kann spiegelsymmetrisch zu einer Ebene umfassend die Koordinatenachse der MR-Anlage verlaufen. Das gekrümmte Schichtprofil kann im Zentrum der Bildgebungsschicht, d.h. bezüglich der lateralen Richtung oder nahe an der Koordinatenachse, orthogonal zu der Koordinatenachse der MR-Anlage verlaufen.

Durch die genannten Ausformungen der Bildgebungsschicht kann ein leichter Übergang von ebenen transversalen Bildgebungsschichten zu der erfindungsgemäßen Bildgebungsschicht erfolgen. Mehrere Bildgebungsschichten entsprechend dieser Formen können zudem leicht in Richtung der Koordinatenachse aneinandergereiht werden, wobei ein Gefäß, welches im Zentrum der Bildgebungsschichten parallel zu der Koordinatenachse verläuft gut abgebildet werden kann. Parabolisch gekrümmte Schichtprofile ermöglichen eine orthogonaleren Inflow des Blutes und damit ein höheres Signal im Peripheriebereich der derart geformten Bildgebungsschichten.

Das Schichtprofil kann zusätzlich durch einen seitlichen Winkel charakterisiert sein, welcher den seitlichen Verlauf des gekrümmten Schichtprofils und die Koordinatenachse der MR-Anlage einschließt. Die Koordinatenachse einerseits, sowie eine Achse, welche im Außenbereich die Bildgebungsschicht einfasst, können den seitlichen Winkel definieren. Der seitliche Winkel kann 30 bis 60 Grad, insbesondere 45 Grad, betragen, wodurch eine gute Darstellung der Bifurkationen in Knie, Arm-Hand, und Becken ermöglicht wird. Der seitliche Winkel kann insbesondere 30 Grad betragen, wodurch eine besonders genaue Darstellung von in 90 Grad zur Koordinatenachse abzweigenden Gefäßen, beispielsweise der abzweigenden Nierenarterien, ermöglicht wird.

Das gekrümmte Schichtprofil kann nur in manuell markierten Regionen der Untersuchungsperson angewendet werden. Dadurch, dass ein herkömmliches Profil in weiteren Untersuchungsregionen verwendet wird, wird die Qualität der angiographischen Bilder verbessert, indem angepasst auf die jeweiligen Gefäßverläufe der passende Schichtverlauf für eine hohe Bildqualität benutzt wird.

Das gekrümmte Schichtprofil kann nur in Regionen der Untersuchungsperson angewendet werden, welche mit Hilfe empirische Patientendaten umfassender Tabellen automatisch markiert wurden.

Empirische Patientendaten können einen durchschnittlichen Patienten beschreiben und insbesondere die Lage von bestimmten Regionen oder Organen, beispielsweise der Nieren, Kniekehlen, oder des Beckens bestimmten Körpergrößenabschnitten zuordnet. Die empirischen Patientendaten können in einer Tabelle, insbesondere in einer Lookup-Tabelle enthalten sein. Das manuelle Markieren von kritischen Untersuchungsregionen ist fehleranfällig, da ein Benutzer entscheiden muss, ob etwa ein Bildfehler, Artefakte oder ein verlässliches Untersuchungsergebnis, etwa ein Gefäßverschluss, vorliegt, und kann durch automatische Markierung mittels empirischer Patientendaten vermieden werden. Die Verwendung von Tabellen, insbesondere von Lookup-Tabellen, beschleunigt die automatische Markierung. Dadurch kann somit eine schnellere Untersuchungszeit und eine höhere Bildqualität erreicht werden, ohne dass ein Benutzer beim Bedienen der MR-Anlage zusätzliche Eingaben betätigen muss.

Das gekrümmte Schichtprofil, insbesondere der seitliche Winkel, kann mit Hilfe empirischer Patientendaten optimiert sein. Die Bestimmung von einem passenden Schichtprofil durch einen Benutzer der MR-Anlage ist fehleranfällig, und kann dadurch nicht immer ein gutes Messergebnis gewährleisten. Insbesondere kann aus Tabellen mit empirischen Patientendaten entnommen werden, welche seitlichen Winkel für unterschiedliche Regionen für gute Bildergebnisse optimal sind, ohne dass eine Bedienperson beim Bedienen der MR-Anlage zusätzliche Eingaben betätigen muss. Es ist insbesondere keine weitere Interaktion des Benutzers, etwa ein Nachfragen oder Bestätigen an einer Benutzerschnittstelle der MR-anlage erforderlich, wodurch Zeitersparnis in der Untersuchungszeit realisiert werden kann.

Das Anregen der Kernmagnetisierung in mindestens einer ersten Bildgebungsschicht kann ein Anregen der Kernmagnetisierung in einer ersten Bildgebungsschicht und in einer weiteren zweiten Bildgebungsschicht umfassen, welche ebenfalls ein gekrümmtes Schichtprofil aufweist. Die erste Bildgebungsschicht und die zweite Bildgebungsschicht können sich teilweise überlappen. Die die erste Bildgebungsschicht und die zweite Bildgebungsschicht können unterschiedliche Schichtprofile aufweisen, oder die gleichen Schichtprofile aufweisen.

Durch die Verwendung von mehreren hintereinander angeordneten, oder sich zumindest teilweise überlappendenden Bildgebungsschichten, wird eine höhere Bildqualität und eine schnellere Untersuchungszeit bereitgestellt. Bei unterschiedlichen Krümmungen der Bildgebungsschichten können diese sich dahingehend ergänzen, dass Details, welche mit der ersten Bildgebungsschicht nicht aufgelöst werden können, mit Hilfe der zweiten Bildgebungsschicht dargestellt werden können.

Weiter können außerhalb der oben beschriebenen markierten Regionen, ebene transversale Schichtprofile angewendet werden, und innerhalb der markierten Regionen die erfindungsgemäß gekrümmten Schichtprofile. Dabei kann der Übergang der Schichtprofile gleitend, oder von Schicht zu Schicht schrittweise in kleinen Schritten, erfolgen. Dadurch wird ohne zusätzlichen Aufwand für den Benutzer eine verbesserte Bildqualität erreicht, ohne dass in den beschriebenen kritischen Regionen weitere hochauflösendere Messsequenzen durchgeführt werden müssen.

Das erfindungsgemäße Verfahren kann zum Erzeugen angiographischer MR-Bilder insbesondere basierend auf dem Quiescent-Intervall Single-Shot (QISS) Verfahren durchgeführt werden, dadurch wird die Robustheit und Qualität dieses Verfahrens gegenüber Inplane-Flow-Artefakten deutlich erhöht.

Gemäß einem weiteren Aspekt der Erfindung wird ein Computerprogrammprodukt bereitgestellt, welches ein Programm umfasst, das direkt in einen Speicher einer MR-Steuereinheit einer MR-Anlage ladbar ist, mit Programm-Mitteln, um die Schritte des Verfahrens entsprechend den unter dem ersten Aspekt der Erfindung beschriebenen Merkmalen auszuführen, wenn das Programm in der MR-Steuereinheit der MR-Anlage ausgeführt wird.

Gemäß einem weiteren Aspekt der Erfindung wird ein elektronisch lesbarer Datenträger mit darauf gespeicherten elektronisch lesbaren Steuerinformationen bereitgestellt, welche derart ausgestaltet sind, dass sie bei Verwendung des Datenträgers in einer MR-Steuereinheit einer MR-Anlage das Verfahren entsprechend den unter dem ersten Aspekt der Erfindung beschriebenen Merkmalen durchführen.

Für das derartige Computerprogrammprodukt sowie für den derartigen elektronischen Datenträger können technische Effekte erzielt werden, die vergleichbar sind mit den technischen Effekten, die für das Verfahren gemäß dem ersten Aspekt obenstehend beschrieben wurden.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### Kurze Beschreibung der Zeichnungen

Die Erfindung wird nachfolgend unter Bezugnahme auf die beiliegenden Zeichnungen näher erläutert.
Figur 1 zeigt schematisch eine MR-Anlage, mit der ein Verfahren zum Erstellen von angiographischen MR-Bildern, welche auf in ein Bildgebungsvolumen einfließenden ungesättigten Spins beruhen, erfindungsgemäß durchgeführt werden kann.
Figur 2 zeigt eine schematische Darstellung eines Übersichtsbildes einer Untersuchungsperson mit gekrümmten Schichtprofilen, gemäß Ausführungsbeispielen der Erfindung.
Figur 3 zeigt schematisch einen Detailausschnitt der Figur 2 im Bereich der Trifurkation des Knies der Untersuchungsperson, gemäß Ausführungsbeispielen der Erfindung.
Figur 4 zeigt eine schematische Darstellung der Niere einer Untersuchungsperson mit gekrümmten Schichtprofilen, gemäß Ausführungsbeispielen der Erfindung.
Figur 5 zeigt ein Flussdiagramm mit den Schritten zum Erstellen von angiographischen MR-Bildern, welche auf in ein Bildgebungsvolumen einfließenden ungesättigten Spins beruhen, gemäß Ausführungsbeispielen der Erfindung.

### Detaillierte Beschreibung

Die vorliegende Erfindung betrifft ein Verfahren zur Gefäßdarstellung mit Hilfe einer MR-Anlage, bei welchem angiographische MR-Bilder erstellt werden, welche auf in ein Bildgebungsvolumen einfließenden ungesättigten Spins beruhen. Bei einer Gefäßdarstellung, welche auf in ein Bildgebungsvolumen einfließenden ungesättigten Spins beruht, wird eine ortsfeste Magnetisierung, welche ein unerwünschtes Hintergrundsignal in einer Bildgebungsschicht erzeugt, durch einen HF-Puls gesättigt. Dabei sind der HF-Puls und das angelegte Magnetfeld derart ausgebildet, dass sie die Hintergrundsignale unterdrücken, ohne die in das Bildgebungsvolumen zufließenden Spins der Gefäßflüssigkeit anzuregen.

Im Sinne der vorliegenden Offenbarung sind Verfahren zum Erstellen von angiographischen MR-Bildern, welche auf in ein Bildgebungsvolumen einfließenden ungesättigten Spins beruhen, inflow-basierte, auf deutsch einflußbasierte, MR-Bildgebungsverfahren, auch genannt Time-of-Flight (ToF) Techniken, oder Einstrom-Techniken. Dabei betrifft das erfindungsgemäße Verfahren insbesondere das QISS (quiescent intervall single shot) Verfahren, weiter MR-Angiographie, insbesondere kontrastmittelfreie MR-Angiographieverfahren.

Im Sinne der vorliegenden Offenbarung werden die Begriffe Bildgebungsschicht, Bildgebungsvolumen, Akquisitionsschicht, oder Schicht synonym verwendet und beschreiben ein Volumen der Untersuchungsperson in der MR-Anlage, in welchem Spins zur Erzeugung von MR-Bilddaten angeregt werden, und aus welchem MR-Signale zum Erzeugen eines MR-Bildes, welches das entsprechende Bildgebungsvolumen darstellt, gemessen werden.

Im Sinne der vorliegenden Offenbarung beschreibt der Begriff Schichtprofil die 3-dimensionale geometrische Form einer Bildgebungsschicht. Ein Schichtprofil ist gekrümmt, wenn die Oberflächen, welche die Bildgebungsschicht einfassen, keine ebenen transversalen Flächen sind, sondern in oder gegen die Richtung der Koordinatenachse gekrümmt sind. Eine vordere Oberfläche der Bildgebungsschicht, welche in Richtung der Koordinatenachse eine erste begrenzende Oberfläche der Bildgebungsschicht darstellt, und eine hintere Oberfläche der Bildgebungsschicht, welche in Richtung der Koordinatenachse eine zweite begrenzende Oberfläche der Bildgebungsschicht darstellt, sind keine flachen, ebenen Flächen, sondern gekrümmte oder gewölbte Flächen. Insbesondere können beide in die gleiche Richtung, sowohl in Richtung als auch gegen die Richtung der Koordinatenachse, gekrümmt sein. Die Bildgebungsschicht kann daher eine gleichmäßige Schichtdicke aufweisen, wenn beide Oberflächen gleich gekrümmt sind, oder eine Variation der Schichtdicke aufweisen, wenn die Oberflächen zwar in die gleiche Richtung, aber unterschiedlich stark gekrümmt sind.

Im Sinne der vorliegenden Offenbarung sind empirische Patientendaten aus einer Vielzahl von Patienten gewonnen und repräsentieren somit durchschnittliche oder statistische Werte von Patienten, d.h. eines durchschnittlichen Patienten. Basierend auf wenigen Kenngrößen, wie etwa Geschlecht, Alter oder Körpergröße, beschreiben die empirischen Patientendaten die Lage von bestimmten Organen oder zu untersuchenden Regionen bezüglich Körperabschnitten des Durchschnittspatienten. Die Daten können in Form von Tabellen vorliegen, so dass ohne weitere Berechnung die örtliche Lage einer Untersuchungsregion in Bezug auf eine Untersuchungsperson bestimmt werden kann.

Erfindungsgemäß ist die Koordinatenachse eine Längsachse eines Patiententunnels der MR-Anlage. Generell umfasst im Sinne der vorliegenden Offenbarung der Begriff Koordinatenachse der MR-Anlage insbesondere eine Haupt-Koordinatenachse der MR-Anlage, zu welcher herkömmlicherweise transversale ebene Bildgebungsschichten in der x-y-Ebene angeregt werden, auch genannt Patientenachse, z-Achse oder Verschiebungsachse des Patiententisches.

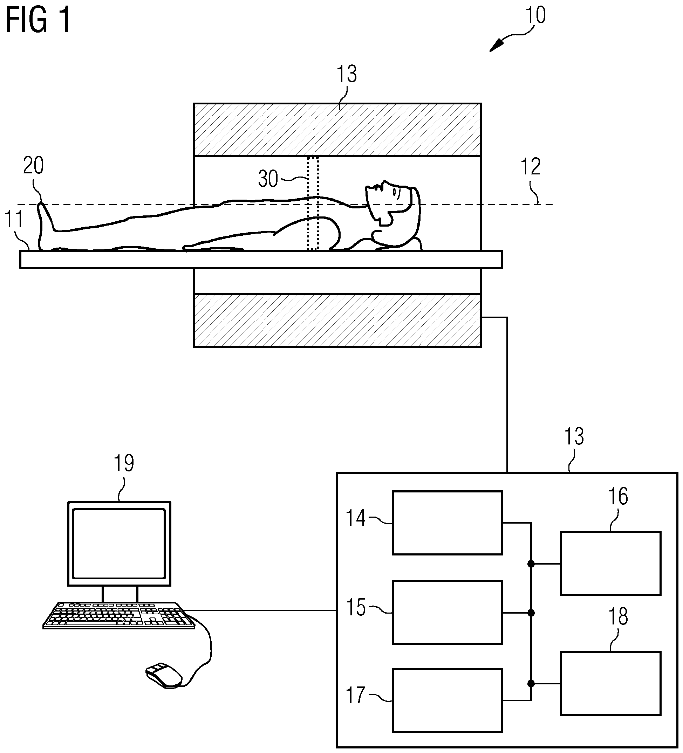

Figur 1 zeigt schematisch eine MR-Anlage 10, mit der ein Verfahren zum Erstellen von angiographischen MR-Bildern, welche auf in ein Bildgebungsvolumen einfließenden ungesättigten Spins beruhen, erfindungsgemäß durchgeführt werden kann.

Eine Untersuchungsperson 20 ist in den Tunnel der Magnetresonanz (MR-) Anlage 10 gefahren. Die MR-Anlage 10 weist einen Magneten 13 zur Erzeugung eines Grundfeldes B0 auf, wobei die auf einer Liege 11 angeordnete Untersuchungsperson 20 entlang einer Koordinatenachse 12 der MR-Anlage 10 in das Zentrum des Magneten 13 verschoben wird, um dort ortscodierte Magnetresonanzsignale aus einem Untersuchungsabschnitt aufzunehmen. Durch Einstrahlen von Hochfrequenzpulsfolgen und Schalten von Magnetfeldgradienten kann die durch das Grundfeld B0 erzeugte Magnetisierung gestört werden, durch Auslenkung Kernspins aus der Gleichgewichtslage gebracht werden, und die bei der Rücckehr in die Gleichgewichtslage in Empfangsspulen induzierten Ströme können in Magnetresonanzsignale umgewandelt werden. Die allgemeine Funktionsweise zum Erstellen von MR-Bildern mittels inflow-basierter MR-Angiographieverfahren und die Detektion der MR-Signale sind dem Fachmann bekannt, sodass auf eine detaillierte Erläuterung hiervon verzichtet wird.

Zur Vorbereitung von MR-Angiographiemessungen werden von der Untersuchungsperson 20 und den Gefäßen 21 sogenannte Übersichtsbilder erzeugt, die beispielsweise mit sagittalen, coronalen oder transversalen flusssensitiven Steady State Bildgebungssequenzen erzeugt werden können.

Die Magnetresonanzanlage weist weiterhin eine MR-Steuereinheit 13 auf, die zur Steuerung der MR-Anlage 10 verwendet wird. Die zentrale MR-Steuereinheit 13, welche derart ausgebildet ist, dass sie die unten beschriebenen Verfahren zum Erstellen von inflow-basierten angiographischen MR-Bildern durchführt, weist eine Gradientensteuerung 14 zur Steuerung und Schaltung der Magnetfeldgradienten auf und eine HF-Steuerung 15 zur Steuerung und Einstrahlung der HF-Pulse zur Auslenkung der Kernspins aus der Gleichgewichtlage. In einer Speichereinheit 16 können beispielsweise die für die Aufnahme der MR-Bilder notwendigen Bildgebungssequenzen abgespeichert werden, sowie alle Programme, die zum Betrieb der MR-Anlage notwendig sind. Eine Aufnahmeeinheit 17 steuert die Bildaufnahme und steuert damit in Abhängigkeit von den gewählten Bildgebungssequenzen die Abfolge der Magnetfeldgradienten und HF-Pulse und die Empfangsintervalle von MR-Signalen. Somit steuert die Aufnahmeeinheit 17 auch die Gradientensteuerung 14 und die HF-Steuerung 15. In einer Recheneinheit 18 können MR-Bilder berechnet werden, die auf einer Anzeige 19 angezeigt werden können, wobei eine Bedienperson über eine Eingabeeinheit die MR-Anlage bedienen kann. Die Speichereinheit 16 kann Bildgebungssequenzen und Programmmodule aufweisen, die bei Ausführung in der Recheneinheit 18 von einem der gezeigten Module, das erfindungsgemäße inflow-basierte angiographische Verfahren durchführen. Die HF-Steuerung 15 kann weiterhin ausgebildet sein, durch gekrümmte Bildgebungsschichten die Erstellung von inflow-basierten angiographischen Bildern zu verbessern, wie nachfolgend im Detail erläutert wird. Insbesondere speichert die Speichereinheit 16 dazu von der MR-Steuereinheit 13 ausführbare Steuerinformationen. Weiter ist die Aufnahmeeinheit 17 derart ausgebildet, dass sie das nachfolgend beschriebene Verfahren zur Gefäßdarstellung durchführen kann.

Die MR-Anlage der Figur 1 ist derart ausgebildet, dass sie bei der Ausführung der Steuerinformationen in der MR-Steuereinheit 13 in einem ersten Schritt die Kernmagnetisierung in mindestens einer ersten Bildgebungsschicht 30 der Untersuchungsperson 20 zum Erzeugen von MR-Signalen angeregt. Die mindestens eine erste Bildgebungsschicht 30 umfasst dabei Gefäße 21, welche durch das bildgebende Verfahren dargestellt werden zumindest teilweise. Die Bildgebungsschicht 30 umfasst dabei insbesondere auch solche Gefäßabschnitte 22 der Gefäße 21, die nicht parallel zu einer Koordinatenachse 12 der MR-Anlage 10 verlaufen, in einem Ausführungsbeispiel auch solche, die einen Verlauf mit hohen orthogonalem Anteil bezüglich der Koordinatenachse 12 der MR-Anlage aufweisen.

In einem weiteren Schritt werden die MR-Signale aus der mindestens einen ersten Bildgebungsschicht 30 zum Erstellen von inflow-basierten angiographischen MR-Bildern der Gefäße 21 empfangen. Dabei ist die MR-Anlage derart ausgebildet, dass die mindestens eine erste Bildgebungsschicht 30 ein gekrümmtes Schichtprofil 31 aufweist.

Die MR-Anlage 10 kann zum Anregen der Kernmagnetisierung insbesondere ein lineares Gradientensystem umfassen, welches in allen drei Raumrichtungen streng lineare Gradientenfelder und Gradientenspulen, aufweist, welche in Kombination mit einer 1,2- oder Mehrkanal-Hochfrequenzanregung zur Anregung der Kernspins mittels gekrümmter Schichtprofile eingesetzt werden. Die MR-Anlage 10 kann dabei insbesondere zum Durchführen des Quiescent-Intervall Single-Shot (QISS) Verfahren ausgebildet sein.

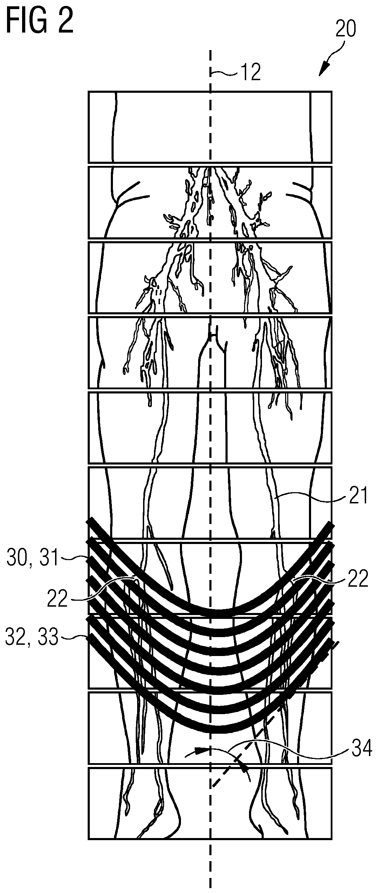

Figur 2 zeigt eine schematische Darstellung eines Übersichtsbildes einer Untersuchungsperson 20 mit gekrümmten Schichtprofilen 31, gemäß Ausführungsbeispielen der Erfindung.

In Figur 2 ist die untere Körperhälfte einer Untersuchungsperson 20 zu sehen. Dabei verläuft die Koordinatenachse 12 der MR-Anlage 10 entlang der Körperlängsachse, oder Patientenachse, der Untersuchungsperson 20. Innerhalb der Untersuchungsperson 20 sind Blutgefäße 21 hell dargestellt. Im Wesentlichen verlaufen die dargestellten Blutgefäße 21 entlang der Koordinatenachse 12 der MR Anlage 10. In einigen kritischen Regionen, beispielsweise der Trifurkation des Knies der Untersuchungsperson 20 verlaufen Gefäßabschnitte 22 orthogonal zu der Koordinatenachse 12, bzw. mit einem hohen orthogonalen Richtungsanteil bezüglich der Koordinatenachse. Daher fließt Blut innerhalb der Gefäßabschnitte 22 in einer orthogonalen Richtung bezüglich der Koordinatenachse 12, in anderen Worten in einem steilen Winkel bezüglich der Koordinatenachse 12.

Weiter sind in Figur 2 Bildgebungsschichten 30 mit gekrümmten Schichtprofilen 31, welche in der erfindungsgemäßen inflow-basierten MR-Bildgebung verwendet werden, abgebildet. Die Bildgebungsschichten 30 weisen in dem Ausführungsbeispiel insbesondere ein parabelförmiges Schichtprofil 31 auf. Das parabelförmige Schichtprofil 31 verläuft im Bereich des Knies der Untersuchungsperson 20 in einem seitlichen Winkel 34 im Bereich von 30 bis 60 Grad, und im Zentrum senkrecht zu der Koordinatenachse 12. Im Bereich der Trifurkation ergibt sich daher über eine begrenzte Strecke ein orthogonaler Einstrom für die Gefäßabschnitte 22, wobei die anderen Gefäße immer noch wenig Inplane Flow generieren.

Auch anders geformte Schichtprofile 31 sind möglich, beispielsweise hyperbelförmige, V-förmige oder pfeilförmige Schichtprofile 31. Dabei sind verschiedene Symmetrien möglich, beispielsweise eine Achsensymmetrie bezüglich der Koordinatenachse 12 der MR Anlage 10, oder eine Spiegelsymmetrie bezüglich einer Ebene umfassend die Koordinatenachse 12. Zwischen der Koordinatenachse und einer an dem seitlichen Verlauf der Schichtprofile 31 anliegenden Achse kann ein seitlicher Winkel 34 eingeschlossen sein. Dieser seitliche Winkel 34 charakterisiert die bildgebenden Schichten 31 im Außenbereich der Bildgebungsschicht 30, d.h. entfernt von der Koordinatenachse. Für senkrecht abzweigende Gefäßabschnitte 22 sind flache seitliche Winkel 34 vorteilhaft. Der seitliche Winkel 34 kann zudem derart bestimmt sein, dass er in Abhängigkeit der abzweigenden Gefäßabschnitte 22 einen möglichst orthogonalen Verlauf der Gefäßabschnitte 22 zu der Bildgebungsschicht 30 ermöglicht. Eine Optimierung des seitlichen Winkels 34 für die gekrümmten Schichtprofile 31 für jede Bildgebungsschicht 30 kann mithilfe von empirischen Patientendaten erfolgen.

Weiter kann auch die Region, in welcher das gekrümmte Schichtprofil 31 angewendet wird, durch die MR Anlage 10 automatisch bestimmt und markiert werden. Dadurch wird eine angiographische Bildgebung automatisch mit einem optimierten Schichtprofil 31 durchgeführt, wobei im Inplane-Flow-Artefakte automatisch vermieden werden. Beispielsweise kann ein Winkel im Bereich von 30 bis 60 Grad, insbesondere 45 Grad, für eine Untersuchung der Trifurkation des Knies mit Hilfe der empirischen Patientendaten bestimmt sein. Eine Bedienperson der MR-Anlage 10 muss in diesem Fall keine Beurteilung eines angiographischen Bildes vornehmen und in keiner Benutzerschnittstelle der MR-Anlage 10 Parameter für eine zusätzliche genauere Untersuchung festlegen, wodurch eine hohe Zeitersparnis und bessere Qualität der angiographischen Bilder ermöglicht wird.

Die Lage der Trifurkation des Knies der Untersuchungsperson 20 kann mithilfe von empirischen Patientendaten automatisch bestimmt werden. Beispielsweise kann das Geschlecht und die Körpergröße der Untersuchungsperson 20 durch eine Bedienperson der MR-Anlage 10 angegeben werden, und in Folge durch die MR-Anlage 10 automatisch bestimmt werden in welchem Körpergrößenabschnitt der Untersuchungsperson 20 sich die Trifurkation des Knies befindet, und diesen Bereich für eine Anwendung der gekrümmten Schichtprofile 31 automatisch markieren. Besonders schnell kann die automatische Markierung durchgeführt werden, wenn derartige empirische Patientendaten in Form von Tabellen vorliegen. Andernfalls kann die Region der Trifurkation auch manuell durch die Bedienperson der MR Anlage 10 markiert werden. Weitere Untersuchungsregionen werden mit herkömmlichen ebenen Bildgebungsschichten untersucht.

Alternativ könnte man zu vorher manuell markierten Regionen oder durch empirischen Tabellen erhobene Distanzen automatisch markierten Abschnitten einen gleitenden Übergang auf dieses gekrümmte Schichtanregungsprofil 31 durchführen, um die Bildgebung speziell kritischer Gefäßabschnitte 22 weiter zu optimieren.

Wie in Figur 2 weiter zu sehen, werden in der angiographischen Untersuchung der Trifurkation des Knies gemäß dem Ausführungsbeispiel eine erste Bildgebungsschicht 30 mit gekrümmtem Schichtprofil 31, eine zweite Bildgebungsschicht 32 mit gekrümmtem Schichtprofil 33, sowie weitere Bildgebungsschichten dargestellt. Die Bildgebungsschichten sind in dem Ausführungsbeispiel gleich geformt. Um eine besonders genaue Bildgebung der Trifurkation bereitzustellen, können die Bildgebungsschichten in einem weiteren nicht dargestellten Ausführungsbeispiel auch unterschiedliche Krümmungen aufweisen. Dadurch kann darauf eingegangen werden, dass Gefäßabschnitte 22 im Bereich der unterschiedlichen Bildgebungsschichten veränderliche Verläufe aufweisen. Die Bildgebungsschichten 30 können sich dabei auch teilweise überlappen, sowie unterschiedliche Schichtdicken aufweisen.

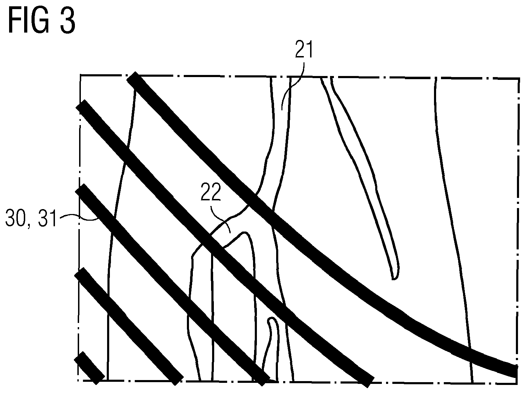

Figur 3 zeigt schematisch einen Detailausschnitt der Figur 2 im Bereich der Trifurkation des Knies der Untersuchungsperson 20, gemäß Ausführungsbeispielen der Erfindung.

Wie in Figur 3 zu sehen, verlaufen die Gefäßabschnitte 22, welche einen hohen orthogonalen Verlauf aufweisen, nicht parallel zu den erfindungsgemäßen Bildgebungsschichten 30, sondern weisen einen senkrechteren Verlauf in Bezug auf die Bildgebungsschichten 30 auf. In den Gefäßabschnitten 22 strömt Blut daher mit einem geringen parallelen Anteil innerhalb der Bildgebungsschichten 30, und mit einem hohen orthogonalen Anteil, daher in einem steilen Winkel in die Bildgebungsschichten 30 hinein. Dadurch werden effektiver ungesättigte Spins in das Bildgebungsvolumen 30 eingebracht, sodass in Inplane-Flow-Artefakte vermieden werden können. Eine Anwendung von parabolisch gekrümmten Profilen bei der Schichtanregung kann somit in dieser kritischen Region der Trifurkation den Bluteinstrom auch für abzweigende Gefäße 22 orthogonaler machen und damit ein höheres Blutsignal erzeugen.

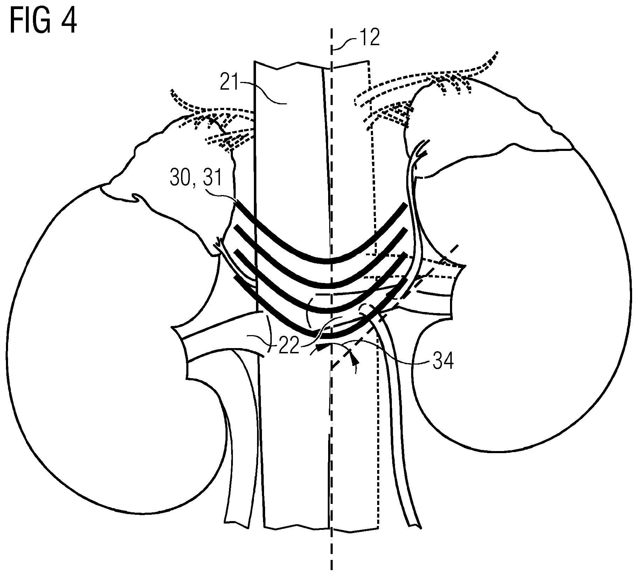

Figur 4 zeigt eine schematische Darstellung der Niere einer Untersuchungsperson 20 mit gekrümmten Schichtprofilen 31, gemäß Ausführungsbeispielen der Erfindung.

In Figur 4 sind sowohl entlang der Koordinatenachse 12 der MR Anlage 10 verlaufende Hauptarterien 21 zu sehen, als auch senkrecht zu den Blutgefäßen 21 nach links und rechts abgehende Gefäßabschnitte 22, welche abzweigende Nierenarterien darstellen. Die in Figur 4 weiter dargestellten Bildgebungsschichten 30 für inflow-basierte angiographische MR-Verfahren, haben mit gekrümmten Schichtprofile 31 mit bogenförmigen Verläufen. Dadurch schneiden sie die zentralen, entlang der Koordinatenachse 12 verlaufenden Arterien 21 senkrecht, aber auch die abzweigende in Nierenarterien 22 verlaufen in einem steilen Winkel zu dem äußeren Verlauf der Bildgebungsschichten 30. Somit können sowohl die zentralen Arterien 21 als auch die abzweigende Nierenarterien 22 durch das gekrümmte Schichtprofil 31 in einem inflow-basierten MR Bildgebungsverfahren ohne Inplane-Flow-Artefakte dargestellt werden.

Alternativ kann eine schräge Schichtführung, d.h. transversal nach coronal/sagittal, durchgeführt werden, was das Problem der Inplane-Flow-Artefakte aber nur auf die entlang der Koordinatenachse 12 verlaufenden Gefäße verlagert. Bei erhöhtem Zeitaufwand können auch mehrere Messungen unterschiedlicher Schichtneigung in kritischen Körperregionen durchgeführt und diese dann fusioniert werden, um das Blutsignal an allen Gefäßabschnitten 22 zu erhöhen.

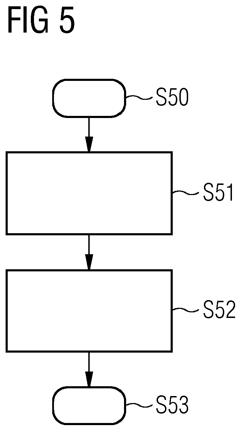

Figur 5 zeigt ein Flussdiagramm mit den Schritten zum Erstellen von angiographischen MR-Bildern, welche auf in ein Bildgebungsvolumen einfließenden ungesättigten Spins beruhen, gemäß Ausführungsbeispielen der Erfindung.

Das Verfahren beginnt in Schritt S50. In Schritt S51 wird die Kernmagnetisierung in mindestens einer ersten Bildgebungsschicht der Untersuchungsperson zum Erzeugen von MR-Signalen angeregt. Die mindestens eine erste Bildgebungsschicht umfasst dabei die Gefäße, welche durch das bildgebende Verfahren dargestellt werden sollen, insbesondere auch Gefäßabschnitte, die nicht parallel zu einer Koordinatenachse der MR-Anlage verlaufen. In Schritt S52 werden die MR-Signale aus der mindestens einen ersten Bildgebungsschicht zum Erstellen von angiographischen MR-Bildern der Gefäße empfangen. Dabei weist die mindestens eine erste Bildgebungsschicht ein gekrümmtes Schichtprofil auf. Das Verfahren endet in Schritt S53.

Zusammenfassend wird ein inflow-basiertes angiographisches MR-Bildgebungsverfahren bereitgestellt, wobei durch gekrümmte Schichtprofile der Bildgebungsschichten auch solche Gefäßabschnitte, die nicht parallel zu einer Koordinatenachse einer MR-Anlage verlaufen, ohne Inplane-Flow-Artefakte dargestellt werden können. Die gekrümmten Schichtprofile können entsprechend empirischer Patientendaten automatisch in bestimmten kritischen Regionen der Untersuchungsperson eingesetzt werden. Insbesondere können die gekrümmten Schichtprofile dabei durch ein Standard-Gradientensystem mit streng linearen Gradientenfeldern in Kombination mit 1,2- oder Mehrkanal-Hochfrequenzanregung erzeugt werden. Dadurch wird ein verbessertes Verfahren zum Erstellen von inflow-basierten angiographischen MR-Bildern bereitgestellt, welches Gefäßabschnitte einer Untersuchungsperson, die nicht parallel zu einer Koordinatenachse einer MR-Anlage verlaufen, zuverlässig und fehlerfrei abbildet. Das Verfahren benötigt dadurch einen geringeren Zeit- und Personalaufwand, und stellt genauere Untersuchungsergebnisse bei geringeren Untersuchungskosten bereit.

## Patentansprüche

1. Verfahren zum Erstellen von angiographischen MR-Bildern, welche auf in ein Bildgebungsvolumen einfließenden ungesättigten Spins beruhen, bei welchem die Signalintensität des stationären Gewebes innerhalb mindestens einer ersten Bildgebungsschicht (30) vor dem Anregen der Kernmagnetisierung mittels eines nichtselektiven Sättigungs-RF-Impulses reduziert wird, wobei Gefäßabschnitte (22) von darzustellenden Gefäßen (21) einer Untersuchungsperson (20) in einer MR-Anlage (10) nicht parallel zu einer Koordinatenachse (12) der MR-Anlage verlaufen, wobei die Koordinatenachse (12) eine Längsachse eines Patiententunnels der MR-Anlage (10) ist, wobei die mindestens eine erste Bildgebungsschicht (30) transversal zu der Koordinatenachse (12) angeregt wird, umfassend folgende Schritte:
- Anregen der Kernmagnetisierung in der mindestens einen ersten Bildgebungsschicht (30) der Untersuchungsperson (20) zum Erzeugen von MR-Signalen; und
- Empfangen der MR-Signale aus der mindestens einen ersten Bildgebungsschicht (30) zum Erstellen von angiographischen MR-Bildern der Gefäße (21);
wobei die mindestens eine erste Bildgebungsschicht (30) ein gekrümmtes Schichtprofil (31) aufweist, welches derart bestimmt ist, dass es im Bereich, in welchem die Gefäßabschnitte (22) die mindestens eine erste Bildgebungsschicht (30) schneiden, einen möglichst orthogonalen Verlauf der Gefäßabschnitte (22) zu der mindestens einen ersten Bildgebungsschicht (30) ermöglicht.

2. Verfahren nach Anspruch 1, wobei das Anregen der Kernmagnetisierung in der mindestens einen ersten Bildgebungsschicht (30) unter Verwendung eines linearen Gradientensystems der MR-Anlage (10), welches streng lineare Gradientenfelder in allen drei Raumrichtungen aufweist, wobei keine nicht-linearen Charakteristiken einer Sendespule berücksichtigt werden, in Kombination mit einer 1,2- oder Mehrkanal-Hochfrequenzanregung durchgeführt wird.

3. Verfahren nach Anspruch 1 oder 2, wobei, wobei das gekrümmte Schichtprofil (31) ein parabelförmiges, hyperbelförmiges, V-förmiges oder pfeilförmiges Schichtprofil ist.

4. Verfahren nach einem der vorhergehenden Ansprüche, wobei das gekrümmte Schichtprofil (31) spiegelsymmetrisch zu einer Ebene umfassend die Koordinatenachse (12) der MR-Anlage ist.

5. Verfahren nach einem der vorhergehenden Ansprüche, wobei das gekrümmte Schichtprofil (31) im Zentrum der Bildgebungsschicht (30) orthogonal zu der Koordinatenachse (12) der MR-Anlage verläuft.

6. Verfahren nach einem der vorhergehenden Ansprüche, wobei das Schichtprofil zusätzlich durch einen seitlichen Winkel (34) charakterisiert ist, welcher durch die Koordinatenachse (12) einerseits, sowie eine Achse, welche die Koordinatenachse schneidet und welche die Bildgebungsschicht (30) entfernt von der Koordinatenachse (12) einfasst, definiert ist.

7. Verfahren nach Anspruch 6, wobei der seitliche Winkel (34) 30-60 Grad, insbesondere 45 Grad, beträgt.

8. Verfahren nach einem der vorhergehenden Ansprüche, wobei das gekrümmte Schichtprofil (31) nur in manuell markierten Regionen der Untersuchungsperson (20) angewendet wird.

9. Verfahren nach einem der vorhergehenden Ansprüche, wobei das gekrümmte Schichtprofil (31) nur in Regionen der Untersuchungsperson (20) angewendet wird, welche mit Hilfe empirische Patientendaten umfassender Tabellen automatisch markiert wurden.

10. Verfahren nach einem der vorhergehenden Ansprüche, wobei das gekrümmte Schichtprofil (31), insbesondere der seitliche Winkel (34), mit Hilfe empirischer Patientendaten optimiert ist.

11. Verfahren nach einem der vorhergehenden Ansprüche, wobei das Anregen der Kernmagnetisierung in der mindestens einen ersten Bildgebungsschicht (30), ein Anregen der Kernmagnetisierung in einer weiteren zweiten Bildgebungsschicht (32) umfasst, welche ebenfalls ein gekrümmtes Schichtprofil (33) aufweist.

12. Verfahren nach Anspruch 11, wobei die erste Bildgebungsschicht (30) und die zweite Bildgebungsschicht (32) sich teilweise überlappen.

13. Verfahren nach einem der Ansprüche 11 oder 12, wobei die erste Bildgebungsschicht (30) und die zweite Bildgebungsschicht (32) unterschiedliche Schichtprofile (31, 33) aufweisen.

14. Verfahren nach einem der vorhergehenden Ansprüche, wobei die angiographischen MR-Bilder mit dem Quiescent-Intervall Single-Shot (QISS) Verfahren erzeugt werden.

15. Computerprogrammprodukt, welches ein Programm umfasst, das direkt in einen Speicher einer MR-Steuereinheit (13) einer MR-Anlage (10) ladbar ist, mit Programm-Mitteln, um die Schritte des Verfahrens nach einem der Ansprüche 1 bis 14 auszuführen, wenn das Programm in der MR-Steuereinheit (13) der MR-Anlage (10) ausgeführt wird und wenn sich eine Untersuchungsperson in der MR-Anlage (10) befindet.

16. Elektronisch lesbarer Datenträger mit darauf gespeicherten elektronisch lesbaren Steuerinformationen, welche ausgestaltet sind, die Schritte des Verfahrens nach einem der Ansprüche 1 bis 14 auszuführen, wenn die Steuerinformationen in einer MR-Steuereinheit einer MR-Anlage ausgeführt werden und wenn sich eine Untersuchungsperson in der MR-Anlage (10) befindet.

## Claims

1. Method for producing angiographic MR images that are based on unsaturated spins flowing into an imaging volume, in which the signal intensity of the stationary tissue within at least one first imaging slice (30) is reduced by a non-selective RF saturation pulse before exciting the nuclear magnetisation, wherein vessel segments (22) of vessels (21) to be represented of a person (20) under examination in an MR system (10) do not run parallel to a coordinate axis (12) of the MR system, wherein the coordinate axis (12) is a longitudinal axis of a patient tunnel of the MR system (10), wherein the at least one first imaging slice (30) is excited transverse to the coordinate axis (12), comprising the following steps:
- exciting the nuclear magnetisation in the at least one first imaging slice (30) of the person (20) under examination in order to generate MR signals; and
- receiving the MR signals from the at least one first imaging slice (30) in order to produce angiographic MR images of the vessels (21);
wherein said at least one first imaging slice (30) has a curved slice profile (31), which is defined such that, in the region in which the vessel segments (22) intersect the at least one first imaging slice (30), it allows the path of the vessel segments (22) to be as orthogonal as possible to the at least one first imaging slice (30).

2. Method according to claim 1, wherein the nuclear magnetisation in the at least one first imaging slice (30) is excited using a linear gradient system of the MR system (10), which linear gradient system has strongly linear gradient fields in all three spatial directions, wherein non-linear characteristics of a transmit coil are not taken into account, in combination with 1-channel, 2-channel or multichannel radiofrequency excitation.

3. Method according to claim 1 or 2, wherein the curved slice profile (31) is a parabolic, hyperbolic, V-shaped or arrow-shaped slice profile.

4. Method according to one of the preceding claims, wherein the curved slice profile (31) is mirror-symmetric about a plane comprising the coordinate axis (12) of the MR system.

5. Method according to one of the preceding claims, wherein in the centre of the imaging slice (30), the curved slice profile (31) runs orthogonal to the coordinate axis (12) of the MR system.

6. Method according to one of the preceding claims, wherein the slice profile is also **characterised by** a lateral angle (34), which is defined on one side by the coordinate axis (12) as well as an axis, which intersects the coordinate axis and which borders the imaging slice (30) away from the coordinate axis (12).

7. Method according to claim 6, wherein the lateral angle (34) equals 30-60 degrees, in particular 45 degrees.

8. Method according to one of the preceding claims, wherein the curved slice profile (31) is employed only in manually labelled regions of the person (20) under examination.

9. Method according to one of the preceding claims, wherein the curved slice profile (31) is employed only in regions of the person (20) under examination that have been labelled automatically using tables comprising empirical patient data.

10. Method according to one of the preceding claims, wherein empirical patient data is used to optimise the curved slice profile (31), in particular the lateral angle (34).

11. Method according to one of the preceding claims, wherein exciting the nuclear magnetisation in the at least a first imaging slice (30) involves exciting the nuclear magnetisation in an additional, second imaging slice (32), which likewise has a curved slice profile (33).

12. Method according to claim 11, wherein the first imaging slice (30) and the second imaging slice (32) overlap partially.

13. Method according to claim 11 or 12, wherein the first imaging slice (30) and the second imaging slice (32) have different slice profiles (31, 33).

14. Method according to one of the preceding claims, wherein the angiographic MR images are generated using the quiescent interval single shot (QISS) method.

15. Computer program product which comprises a program that can be loaded directly into a memory of an MR control unit (13) of an MR system (10), and which has program means in order to perform the steps of the method according to one of claims 1 to 14 when the program is executed in the MR control unit (13) of the MR system (10) and when a person under examination is located in the MR system (10).

16. Electronically readable data storage medium comprising electronically readable control information stored thereon, which information is designed to perform the steps of the method according to one of claims 1 to 14 when the control information is executed in an MR control unit of an MR system and when a person under examination is located in the MR system (10).

## Revendications

1. Procédé d'établissement d'images RM angiographiques, qui repose sur des spins insaturés entrant dans un volume d'imagerie, dans lequel on réduit, au moyen d'une impulsion RF de saturation non sélective avant l'excitation de la magnétisation nucléaire,, l'intensité du signal du tissu stationnaire au sein d'au moins une première tranche (30) d'imagerie, dans lequel des tronçons (22) de vaisseau (21) à représenter d'une personne (20) à examiner, dans une installation (10) RM, ne s'étendent pas parallèlement à un axe (12) de coordonnée de l'installation RM, l'axe (12) de coordonnée étant un axe longitudinal d'un tunnel pour patient de l'installation (10) RM, dans lequel on excite la au moins une première tranche (30) transversalement à l'axe (12) de coordonnée, comprenant les stades suivants :
- excitation de la magnétisation nucléaire dans la au moins un première tranche (30) d'imagerie de la personne (20) à examiner pour la production de signaux RM ;
- réception de signaux RM provenant de la au moins une première tranche (30) d'imagerie pour l'établissement d'images RM angiographique des vaisseaux (21) ;
- dans lequel la au moins une première tranche (30) d'imagerie a un profil (31) de tranche incurvé, qui est déterminé de manière à ce que, dans la partie dans laquelle les tronçons (22) de faisceau coupent la au moins une première tranche (30) d'imagerie, soit possible un tracé le plus orthogonal possible des tronçons (22) de vaisseau par rapport à la au moins une première tranche (30) d'imagerie.

2. Procédé suivant la revendication 1, dans lequel on effectue, en combinaison avec une excitation de haute fréquence à un, deux ou plusieurs canaux, l'excitation de la magnétisation nucléaire dans la au moins une première tranche (30) d'imagerie, en utilisant un système de gradient linéaire de l'installation (10) RM, qui a des champs de gradient strictement linéaires dans toutes les trois directions de l'espace, aucune caractéristique non-linéaire d'une bobine d'émission n'étant prise en compte.

3. Procédé suivant la revendication 1 ou 2, dans lequel le profil (31) de tranche incurvé est un profil de tranche parabolique, hyperbolique, en forme de V ou en forme de flèche.

4. Procédé suivant l'une des revendications précédentes, dans lequel le profil (31) de tranche incurvé est symétrique comme en un miroir, par rapport à un plan comprenant l'axe (12) de coordonnées de l'installation RM.

5. Procédé suivant l'une des revendications précédentes, dans lequel le profil (31) de tranche incurvé s'étend au centre de la tranche (30) d'imagerie orthogonalement à l'axe (12) de coordonnée de l'installation RM.

6. Procédé suivant l'une des revendications précédentes, dans lequel le profil de tranche est **caractérisé en outre par** un angle (34) latéral, qui est défini par l'axe (12) de coordonnée d'une part, ainsi que par un axe, qui coupe l'axe de coordonnée et qui borde la tranche (30) d'imagerie loin de l'axe (12) de coordonnée.

7. Procédé suivant la revendication 6, dans lequel l'angle (34) latéral va de 30 à 60 degrés, en étant notamment de 45 degrés.

8. Procédé suivant l'une des revendications précédentes, dans lequel on n'applique le profil (31) de tranche incurvé que dans des régions repérées manuellement de la personne (20) à examiner.

9. Procédé suivant l'une des revendications précédentes, dans lequel on n'applique le profil (31) de tranche incurvé que dans des régions de la personne (20) à examiner, qui ont été repérées automatiquement à l'aide de tables comprenant des données empiriques de patients.

10. Procédé suivant l'une des revendications précédentes, dans lequel le profil (31) de tranche incurvé, notamment l'angle (34) latéral, est optimisé à l'aide de données empiriques de patients.

11. Procédé suivant l'une des revendications précédentes, dans lequel l'excitation de la magnétisation nucléaire dans la au moins une première tranche (30) d'imagerie comprend une excitation de la magnétisation nucléaire dans au moins une autre deuxième tranche (32) d'imagerie, qui a également un profil (33) de tranche incurvé.

12. Procédé suivant la revendication 11, dans lequel la première tranche (30) et la deuxième tranche (32) d'imagerie se chevauchent en partie.

13. Procédé suivant l'une des revendications 11 ou 12, dans lequel la première tranche (30) d'imagerie et la deuxième tranche (32) d'imagerie ont des profils (31, 33) de tranche différents.

14. Procédé suivant l'une des revendications précédentes, dans lequel on produit les images RM angiographiques par le procédé Quiescent Intervall Single Shot (QISS).

15. Produit de programme d'ordinateur, qui comprend un programme, qui peut être chargé directement dans une mémoire d'une unité (13) de commande RM d'une installation (10) RM, comprenant des moyens de programme pour effectuer les stades du procédé suivant l'une des revendications 1 à 14, lorsque le programme est réalisé dans l'unité (13) de commande RM de l'installation (10) RM et lorsqu'une personne à examiner se trouve dans l'installation (10) RM.

16. Support de données déchiffrable électroniquement, sur lequel sont mis en mémoire des informations de commande déchiffrables électroniquement, qui sont conformées pour effectuer les stades du procédé suivant l'une des revendications 1 à 14 lorsque les informations de commande sont réalisées dans une unité de commande RM d'une installation RM et lorsqu'une personne à examiner se trouve dans l'installation (10) RM.
